# EUROPÄISCHE PATENTANMELDUNG

(11) **EP 4 417 698 A1**
(43) Veröffentlichungstag der Anmeldung: **21.08.2024**
(21) Anmeldenummer: 23156866.8
(22) Anmeldetag: 15.02.2023
(51) Int. Cl.: C12P 13/00, C12P 13/04, C12R 1/38, C12R 1/185, C12R 1/15, C12N 1/20

(54) **GLYKIERUNG VON AMINOBENZOESÄURE ZUR VERBESSERUNG DER EFFIZIENZ MIKROBIELLER VERFAHREN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung beruht auf der Entdeckung, dass glykierte Aminobenzoesäure (AB) für mikrobielle Zellen deutlich weniger toxisch ist als freie AB. Deswegen werden Fermentationsmedien und Verfahren beschrieben, die eine Glykierung von AB ermöglichen und so die Effizienz mikrobieller Verfahren, bei denen AB im Medium vorliegt oder entsteht, erhöhen.

## Beschreibung

Die vorliegende Erfindung beruht auf der Entdeckung, dass glykierte Aminobenzoesäure (AB) für mikrobielle Zellen deutlich weniger toxisch ist als freie AB. Deswegen werden Fermentationsmedien und Verfahren beschrieben, die eine Glykierung von AB ermöglichen und so die Effizienz mikrobieller Verfahren, bei denen AB im Medium vorliegt oder entsteht, erhöhen.

Die Herstellung von ortho-Aminobenzoesäure (oAB) durch mikrobielle Fermentationen ist aus dem Stand der Technik gut bekannt (z.B. Balderas-Hernandez et al. (2009) "Metabolic engineering for improving anthranilate synthesis from glucose in Escherichia coli", Microbial Cell Factories 8: 19, WO 2015/124687 und WO 2017/102853). Auch die fermentative Herstellung von para-Aminobenzoesäure (pAB) wurde beschrieben (Kubota et al. (2016) "Production of poro-aminobenzoate by genetically engineered Corynebacterium glutamicum and non-biological formation of an N-glucosyl byproduct", Metab. Eng. 38: 322-330). In dieser Studie wurde auch beobachtet, dass pAB in Anwesenheit von Glucose glykiert wurde. Da die Studie auf ein effizientes Verfahren zur fermentativen Herstellung von pAB abzielte, wurde die Bildung dieser Verbindung als unerwünscht betrachtet.

In den vorveröffentlichten Studien wurde festgestellt, dass pAB und oAB als Fermentationsprodukte für die produzierenden Mikroorganismen bereits in solchen Konzentrationen toxisch sein können, die im Rahmen eines ökonomisch sinnvollen Produktionsverfahrens im Fermentationsmedium erreicht werden müssen (WO 2015/124687 und Kubota, 2016). Somit ist die Wirtschaftlichkeit einer derartigen Herstellung von AB durch die erreichbaren Produktkonzentrationen - und damit die erreichbare Raum-Zeit-Ausbeute - beschränkt. Deswegen ist es wünschenswert, Wege zu finden, die eine Anwesenheit höherer AB-Konzentrationen in einem Fermentationsmedium ermöglichen, ohne dass dadurch die Stoffwechselaktivität der AB-produzierenden Mikroorganismen beeinträchtigt wird. Im Stand der Technik werden zur Verringerung der Toxizität von oAB und pAB bestimmte Zusammensetzungen der verwendeten Fermentationsmedien vorgeschlagen. Die Abwesenheit von Ammonium-Ionen (WO 2019/073035) oder die Anwesenheit von Alkali-Ionen (WO 2019/073033) kann die Empfindlichkeit von Mikroorganismen gegenüber oAB und pAB absenken. Trotzdem gibt es einen Bedarf nach weiteren Methoden zum Schutz der Mikroorganismen vor den Auswirkungen hoher AB-Konzentrationen.

In der vorliegenden Anmeldung zugrunde liegenden Studie wurde überraschend gefunden, dass die Glykierung von AB ihre Toxizität deutlich verringern kann. Eine Glykierung von AB in wässriger Lösung erfolgt spontan, wenn reduzierender Zucker anwesend sind.

Deswegen betrifft die vorliegende Erfindung in einer ersten Ausführungsform eine wässrige Zusammensetzung enthaltend eine Gesamtmenge an Aminobenzoesäure (AB) von wenigstens 40 g/l, wobei wenigstens 2 g/l dieser Gesamtmenge als glykierte AB (gly-AB) vorliegen.

Bevorzugt liegen wenigstens 3 g/l, stärker bevorzugt wenigstens 4 g/l, noch stärker bevorzugt wenigstens 6 g/l und am stärksten bevorzugt wenigstens 8 g/l der Gesamtmenge von AB als glykierte AB vor.

In einer bevorzugten Ausführungsform der vorliegenden Erfindung liegen wenigstens 7,5 % stärker bevorzugt wenigstens 15 % und am stärksten bevorzugt wenigstens 20 % der Gesamtmenge an AB als glykierte AB vor.

### Wässrige Zusammensetzung

Der Begriff "wässrige Zusammensetzung" bezeichnet ein Gemisch, das die oben genannten Komponenten mit Wasser als Lösungsmittel enthält. Die Anwesenheit organischer Lösungsmittel ist bevorzugt auf höchstens 5 Gew.-%, stärker bevorzugt höchstens 2 Gew.-%, noch stärker bevorzugt höchstens 1 Gew.-% und am stärksten bevorzugt höchstens 0,5 Gew.-%, jeweils bezogen auf die Gesamtmenge aller Lösungsmittel, beschränkt. Dies bedeutet umgekehrt, dass der Wasseranteil bezogen auf die Gesamtmenge aller Lösungsmittel wenigstens 95 Gew.-%, stärker bevorzugt wenigstens 98 Gew.-%, noch stärker bevorzugt wenigstens 99 Gew.-% und am stärksten bevorzugt wenigstens 99,5 Gew.-% beträgt.

Bei der wässrigen Zusammensetzung handelt es sich bevorzugt um ein Nährmedium, das eine metabolische Aktivität der hierin optional vorliegenden prokaryotischen Zellen ermöglicht. Deswegen enthält die wässrige Zusammensetzung in einer bevorzugten Ausführungsform der vorliegenden Erfindung für die verwendeten prokaryotischen Zellen verwertbare Quellen anorganischer Nährstoffe, insbesondere Stickstoff, Schwefel und Phosphor sowie die von ihnen benötigten Spurenelemente. Je nach Art der prokaryotischen Zellen kann der Zusatz von Vitaminen und/oder komplexen Medienbestandteilen wie z.B. Pepton oder Hefeextrakt sinnvoll sein. Diese komplexen Medienbestandteile können gleichzeitig auch als Stickstoff-, Schwefel- und/oder Phosphorquellen dienen. Die Begriffe "Stickstoffquelle", "Schwefelquelle" und "Phosphorquelle" bezeichnen deswegen nicht zwingend getrennte chemische Verbindungen, sondern können auch unterschiedliche Funktionen einer Verbindung kennzeichnen.

Darüber hinaus enthält die wässrige Zusammensetzung vorzugsweise wenigstens ein fermentierbares Substrat, das die betreffenden prokaryotischen Zellen als Energie- und Kohlenstoffquelle nutzen können. In einer besonders bevorzugten Ausführungsform der vorliegenden Erfindung handelt es sich bei dem fermentierbaren Substrat um einen der weiter unten in dieser Anmeldung definierten reduzierenden Zucker insbesondere um einen Zucker ausgewählt aus der Gruppe bestehend aus Glucose, Fructose, Mannose, Galactose, Maltose, Lactose und Saccharose.

Besonders geeignete Nährmedien sind in den in dieser Anmeldung enthaltenen Ausführungsbeispielen beschrieben. Grundsätzlich ist der Fachmann aber in der Lage, auf Grundlage der bekannten physiologischen Anforderungen eines Mikroorganismus geeignete Nährmedien auszuwählen.

### Aminobenzoesäure

Der Begriff "Aminobenzoesäure" (AB) ist dem Fachmann bekannt. Er bezeichnet ein Derivat der Benzoesäure, das durch die Anwesenheit einer am Phenylring gebundenen Aminogruppe gekennzeichnet ist. Der Begriff Aminobenzoesäure umfasst die beiden bekannten Strukturisomere, die sich durch die Stellung der Aminogruppe relativ zur Carboxylgruppe unterscheiden: ortho-Aminobenzoesäure (oAB) und para-Aminobenzoesäure (pAB). Wenn in dieser Anmeldung eines der beiden Isomere spezifisch gemeint ist, wird es mit einem der vorgenannten Begriffe bezeichnet. Bei den vorgenannten Verbindungen handelt es sich um Moleküle, die durch Protonierung der Aminogruppe positiv geladen, durch Deprotonierung der Aminogruppe negativ geladen und am isoelektrischen Punkt mit neutraler Gesamtladung vorliegen können. Soweit im Einzelfall nicht anders kenntlich gemacht bezeichnen die Begriffe AB, oAB und pAB Moleküle aller Ladungszustände.

In dieser Anmeldung werden nachfolgend die Begriffe "freie AB", "freie oAB" und "freie pAB" verwendet, wenn kenntlich gemacht werden soll, dass diese ohne chemische Modifikation, insbesondere nicht glykiert, vorliegen. Umgekehrt bezeichnen die Begriffe "Gesamt-AB", "GesamtoAB" und "Gesamt-pAB" jeweils die Summe der vorliegende Moleküle mit der entsprechenden Grundstruktur unabhängig davon, ob sie chemisch durch eine Glykierung modifiziert sind oder nicht.

### Glykierte AB

Der Begriff "Glykierung" bezeichnet die kovalente Bindung eines Zuckers an die Aminogruppe der AB. Dies geschieht vorzugsweise durch Reaktion der Aldehydfunktion eines reduzierenden Zuckers mit der Aminogruppe, wobei Wasser abgespalten wird. Diese Reaktion kann enzymkatalysiert und ohne Beteiligung von Enzymen rein chemisch stattfinden. Die Reaktion ist in wässriger Lösung reversibel und pH-abhängig, so dass die entstandene glykierte AB, oAB oder pAB durch Hydrolyse wieder in die Ausgangsstoffe gespalten werden kann. Aus glykierter AB entstehen dann wieder freie AB und der bei der Glykierung gebundene Zucker.

### Reduzierender Zucker

In einer bevorzugten Ausführungsform der vorliegenden Erfindung enthält die wässrige Zusammensetzung wenigstens einen reduzierenden Zucker. Das molare Verhältnis der Gesamtmenge der Aldehydgruppen des reduzierenden Zuckers zu Gesamt-AB beträgt dabei wenigstens 0,3 : 1,0, bevorzugt wenigstens 0,4 : 1 und noch stärker bevorzugt wenigstens 0,5 : 1.

Da der reduzierende Zucker durch die Glykierung an die AB gebunden wird, muss auch bei der Angabe der Zuckermengen oder -Konzentrationen zwischen dem Gesamtzucker und dem freien Zucker differenziert werden. Der Gesamtzucker bzw. die Gesamtmenge gibt die Summe von frei in der wässrigen Zusammensetzung vorliegendem und durch Glykierung an AB gebundenem reduzierendem Zucker an. Wenn hingegen von "freiem reduzierenden Zucker" die Rede ist, so ist der Anteil des Zuckers gemeint, der nicht an AB gebunden ist und frei in der Zusammensetzung vorliegt.

Der Begriff "reduzierender Zucker" bezeichnet alle Zucker, die über eine Aldehydfunktion verfügen und deswegen in der Lage sind, freie AB zu glykieren. Erfindungsgemäß können sowohl Einfachzucker, als auch mehrwertige Zucker eingesetzt werden, solange sie über eine Aldehydfunktion verfügen. Bevorzugte Einfachzucker sind Glucose, Fructose, Xylose, Arabinose, Mannose und Galactose. Bevorzugte mehrwertige Zucker sind Maltose, Lactose, Saccharose und Cellobiose. Besonders bevorzugte reduzierende Zucker sind Glucose, Xylose, Arabinose und Cellobiose. Ganz besonders bevorzugt ist Glucose. Reduzierende Zucker, die von *Corynebacterium* nicht als energie- oder Kohlenstoffquelle oder zur Bildung eines Zielprodukts verwendet werden können sind Xylose und Cellobiose.

### Prokaryotische Zellen

Die wässrige Zusammensetzung enthält prokaryotische Zellen.

In einer Ausführungsform enthält die wässrige Zusammensetzung eine Gesamtmenge Aminobenzoesäure (AB) von wenigstens 40 g/l und mehrere Zellen des Genus *Corynebacterium.*

In einer alternativen Ausführungsform enthält die wässrige Zusammensetzung eine Gesamtmenge Aminobenzoesäure (AB) von wenigstens 5 g/l und mehrere Zellen des Genus *Escherichia* oder *Pseudomonas.*

Die Zelldichte an prokaryotischen Zellen beträgt vorzugsweise wenigstens 10⁴ Zellen pro ml. In einer weiteren bevorzugten Ausführungsform liegen die prokaryotischen Zellen in einer Dichte von 2 g bis 70 g Trockenmasse pro Liter vor, stärker bevorzugt zwischen 10 g und 30 g Trockenmasse pro Liter.

Unabhängig von der Art der in der wässrigen Zusammensetzung vorliegenden prokaryotischen Zellen sind diese in einer bevorzugten Ausführungsform der vorliegenden Erfindung fähig, ein fermentierbares Substrat zu AB, besonders bevorzugt zu oAB, umzusetzen.

Zellen die über diese Fähigkeit verfügen, sind vorzugsweise durch die nachfolgend definierte physiologische Modifikation (i) gegenüber dem Wildtyp gekennzeichnet. In einer bevorzugten Ausführungsform der vorliegenden Erfindung ist die Zelle zusätzlich durch wenigstens eine der Modifikationen (iii), (iv) und (v) gekennzeichnet. Eine reduzierte Aktivität der Phosphoenolpyruvat Carboxylase wie in (ii) definiert charakterisiert die Zelle in einer besonders bevorzugten Ausführungsform zusätzlich. Bei diesen Zellen kann es sich um alle erfindungsgemäß geeigneten prokaryotischen Zellen handeln, da der Shikimat-Weg, der hier genutzt wird, phylogenetisch stark konserviert ist. Bevorzugt handelt es sich aber um Zellen des Genus *Corynebacterium,* bevorzugt *Corynebacterium glutamicum* und besonders bevorzugt *Corynebacterium glutamicum* ATCC 13032.
(i) Eine gegenüber dem jeweiligen Wildtyp reduzierte Aktivität der Anthranilat-Phosphoribosyltransferase, wobei aber eine Restaktivät vorhanden sein muss. Die Restaktivität liegt vorzugsweise zwischen 10 % und 60 %, stärker bevorzugt zwischen 20 % und 50 % der im jeweiligen Wildtyp nativ vorhandenen Aktivität. Dies wird vorzugsweise durch eine gegenüber dem Wildtyp reduzierte Expression des Gens für die Anthranilat-Phosphoribosyltransferase (trpD) erreicht, wobei die Expression aber nicht vollständig unterbunden ist. Dies erfolgt bevorzugt durch Einsatz einer Promotorsequenz, die gegenüber der endogen vorhandenen Promotorsequenz eine geringere Transkriptionsaktivität aufweist oder durch eine Modifikation des Abstands der Ribosomenbindestelle zum Start-Codon des trpD-Gens oder durch eine Veränderung des Start-Codons selbst. In einer bevorzugten Ausführungsform der vorliegenden Erfindung erfolgt die Reduzierung der Aktivität der Anthranilat-Phosphoribosyltransferase durch Deletion oder Inaktivierung des Gens für die endogene Anthranilat-Phosphoribosyltransferase (trpD) und den Ersatz dieses Gens durch ein Gen für eine Anthranilat-Phosphoribosyltransferase mit einer veränderten ribosomalen Bindestelle und optional einem veränderten Start-Codon wie in SEQ ID NO. 1 oder 2, bevorzugt SEQ ID NO. 2 definiert. Die Aminosäuresequenz der Anthranilat-Phosphoribosyltransferase entspricht vorzugsweise der endogenen Anthranilat-Phosphoribosyltransferase, besonders bevorzugt wird sie durch SEQ ID NO. 3 oder eine Variante davon definiert.
(ii) Deletion oder Inaktivierung des Gens, das für die Phosphoenolpyruvat-Carboxylase wie in SEQ ID NO. 4 definiert kodiert. Dies kann auf alle dem Fachmann geläufigen Weisen geschehen, vorzugsweise durch Deletion des Gens oder einer Teilsequenz davon, durch Einführung wenigstens eines Stop-Codons oder durch Deletion oder Inaktivierung der Promotorsequenz. Besonders bevorzugt ist die Deletion wenigstens eines Teils der proteinkodierenden Sequenz des Gens (SEQ ID NO. 5).
(iii) Erhöhte Aktivität der Shikimat-Kinase. Dies erfolgt vorzugsweise durch erhöhte Expression eines entsprechenden Enzyms. In einer Ausführungsform der vorliegenden Erfindung erfolgt die Steigerung der Aktivität durch erhöhte Expression des Gens für die endogen vorhandene Shikimat-Kinase wie in SEQ ID NO. 6 definiert oder einer Variante davon. In einer anderen bevorzugten Ausführungsform erfolgt dies durch Expression einer exogenen Shikimat-Kinase, bevorzugt wie in SEQ ID NO. 7 definiert oder einer Variante davon. Eine verstärkte Expression eines Genes kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Ein besonders bevorzugter Promotor für die Expression von Fremdgenen oder die verstärkte Expression endogener Gene ist ptuf wie in SEQ ID NO. 8 definiert.
(iv) Erhöhte Aktivität der 3-Phosphoshikimat-1-carboxyvinyltransferase und Chorismat-Synthase. Bevorzugt haben diese Enzyme eine Aminosäuresequenz wie in SEQ ID NO 9 oder einer Variante davon und SEQ ID NO. 10 oder einer Variante davon definiert. Dies erfolgt bevorzugt durch die Einführung zusätzlicher Kopien der für diese Enzyme kodierenden Gene in den Mikroorganismus. Bevorzugt wird der ptuf-Promotor zur Steuerung der Expression verwendet.
(v) Anwesenheit einer 3-Desoxyarabinoheptulosanat-7-phosphat-Synthase (DAHP-Synthase), die "feedback-resistent" ist, d.h. nicht durch ihr Produkt oder durch ein aus dem Produkt entstehendes Produkt gehemmt wird. Bevorzugt ist ein Enzym mit der in SEQ ID NO. 11 definierten Aminosäuresequenz oder eine Variante davon.

In einer bevorzugten Ausführungsform der vorliegenden Erfindung ist die oben definierte prokaryotische Zelle zusätzlich durch erhöhte Aktivität der Xylose-Isomerase und der Xylulokinase. Diese erhöhte Aktivität erfolgt vorzugsweise durch verstärkte Expression der beiden Gene. Diese verstärkte Expression kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Erfindungsgemäß bevorzugt ist eine erhöhte Expression von Enzymen mit Aminosäuresequenzen wie in SEQ ID NO. 12 bzw. SEQ ID NO. 14 definiert oder Varianten davon.

Bezogen auf die Anthranilat-Phosphoribosyltransferase (SEQ ID NO. 3), Shikimat-Kinase (SEQ ID NO. 6 oder 7), 3-Phosphoshikimat-1-carboxyvinyltransferase (SEQ ID NO. 9), Chorismat-Synthase (SEQ ID NO. 10), Xylose-Isomerase (SEQ ID NO. 12) und die Xylulokinase (SEQ ID NO. 14) bedeutet "Variante" ein Enzym, das durch Hinzufügung, Deletion oder Austausch von bis zu 10 %, bevorzugt bis zu 5 %, der im jeweiligen Enzym enthaltenen Aminosäuren erhalten wird. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Die spezifische Enzymaktivität einer Variante liegt vorzugsweise wenigstens bei 80 % der spezifischen Aktivität des unveränderten Enzyms. Enzymtests zum Nachweis der Aktivität der vorgenannten Enzyme kann der Fachmann der Literatur entnehmen.

Bezogen auf die DAHP-Synthase (SEQ ID NO. 11) bedeutet "Variante" ein Enzym, das durch Hinzufügung, Deletion oder Austausch von bis zu 5 %, bevorzugt bis zu 2 %, der im jeweiligen Enzym enthaltenen Aminosäuren erhalten wird, mit der Maßgabe, dass die Positionen 76 und 211 unverändert bleiben. Es ist weiterhin bevorzugt, dass zusätzlich auch die Positionen 10, 13, 147, 148, 150, 151, 179, 209, 211 und 212 unverändert bleiben. In einer stärker bevorzugten Ausführungsform der vorliegenden Erfindung sind in der Variante zusätzlich auch die Positionen 144, 175 und 215 unverändert. In einer noch stärker bevorzugten Ausführungsform der vorliegenden Erfindung sind zusätzlich zu den vorgenannten Positionen auch die Positionen 92, 97, 165, 186 und 268 unverändert. Der Fachmann versteht, dass sich die vorgenannten Positionen entsprechend verschieben, wenn Aminosäuren deletiert oder eingefügt werden. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Es ist besonders bevorzugt, dass auch die Variante der DAHP-Synthase eine entsprechende Enzymaktivität besitzt. Besonders bevorzugt liegt die spezifische Enzymaktivität der Variante bei wenigstens 80 % der spezifischen Aktivität der unveränderten DAHP-Synthase gemäß SEQ ID NO. 11.

### Metabolische Aktivität

In einer bevorzugten Ausführungsform der vorliegenden Erfindung sind die in der wässrigen Zusammensetzung vorliegenden prokaryotischen Zellen metabolisch aktiv. Der Begriff "metabolische Aktivität" bezeichnet einen Zustand der prokaryotischen Zellen, in dem sie ein fermentierbares Substrat aufnehmen und zur Aufrechterhaltung ihrer Lebensfunktionen biochemisch umsetzen.

In einer bevorzugten Ausführungsform der vorliegenden Erfindung wird die metabolische Aktivität durch Wachstum definiert, entweder als Zunahme der Zellmenge oder als Zunahme der Biotrockenmasse der wässrigen Zusammensetzung. Bei konstantem Volumen der wässrigen Zusammensetzung bedeutet dies Zunahme der Zelldichte bzw. der Biotrockenmasse pro Volumeneinheit. Bei steigendem Volumen, insbesondere durch Zuführung frischer Substratlösung, oder bei teilweisem Austausch der wässrigen Zusammensetzung sind diese Veränderungen bei der Berechnung von Zellmenge bzw. Biotrockenmasse zu berücksichtigen. Zellmenge bzw. Biotrockenmasse werden bevorzugt über die Veränderung der Lichtabsorption des Kulturmediums bei einer Wellenlänge von 600 nm oder durch Bestimmung der Rückstreuung bestimmt. Letzteres erfolgt vorzugsweise bei einer Wellenlänge von 620 nm. Hierbei kann der Umrechnungsfaktor zwischen Biotrockenmasse bzw. Zelldichte und Absorption durch dem Fachmann geläufige Methoden bestimmt werden. In einer stärker bevorzugten Ausführungsform wird die metabolische Aktivität durch den Verbrauch des wenigstens einen fermentierbaren Substrats in der wässrigen Zusammensetzung definiert.

Die dieser Anmeldung zugrundeliegende Studie hat gezeigt, dass die Wachstumsrate prokaryotischer Zellen in Anwesenheit einer definierten Konzentration glykierter AB um wenigstens 30 % höher ist als in Anwesenheit der gleichen Konzentration des Natriumsalzes der freien AB. Für den Effekt der erfindungsgemäßen wässrigen Zusammensetzung ist dabei zu berücksichtigen, dass die darin enthaltene AB im Regelfall nicht vollständig glykiert vorliegen wird, so dass der Effekt bei der realen Anwendung der erfindungsgemäßen Zusammensetzung auch geringer ausfallen kann.

In einer anderen bevorzugten Ausführungsform der vorliegenden Erfindung ist die metabolische Aktivität als Produktion von Aminobenzoesäure definiert.

### Toxizität von AB

Der Begriff "Toxizität von AB" bezeichnet den nachteiligen Einfluss, den Aminobenzoesäure ab einer bestimmten Konzentration auf die metabolische Aktivität von prokaryotischen Zellen hat. Ab einem bestimmten Schwellenwert, der vom verwendeten Organismus und auch von den verwendeten Medienbestandteilen (siehe WO 2019/073035 und WO 2019/073033) abhängt, wird die Aktivität der prokaryotischen Zellen durch steigende AB-Konzentrationen zunehmend gehemmt. Dies führt im Extremfall zum Absterben der Zellen.

In der der vorliegenden Patentanmeldung zugrundeliegenden Studie wurde nun überraschend gefunden, dass die spezifische Toxizität glykierter AB geringer ist als die spezifische Toxizität freier AB, d.h. eine gegebene molare Konzentration freier AB im Nährmedium hemmt die metabolische Aktivität einer prokaryotischen Zelle stärker als dieselbe molare Konzentration glykierter AB. Deswegen hat es sich als vorteilhaft erwiesen, in einem Nährmedium Bedingungen zu schaffen, die eine Glykierung der freien AB fördern. Aufgrund des Einflusses von anderen Medienbestandteilen auf die Toxizität von AB wird dieser Vergleich vorzugsweise mit Medien durchgeführt, die sich nur in den Konzentrationen von freier und glykierter AB unterscheiden und insbesondere ansonsten gleiche Konzentrationen von Natrium- und Ammoniumionen aufweisen.

### Verwendung der wässrigen Zusammensetzung

In einer weiteren Ausführungsform betrifft die vorliegende Anmeldung die Verwendung der oben definierten wässrigen Zusammensetzung zur Kultivierung prokaryotischer Zellen.

Alle oben für die wässrige Zusammensetzung gegebenen Definition gelten auch für diese Ausführungsform, soweit nicht explizit anders angegeben.

Der Begriff "Kultivierung", wenn er auf prokaryotische Zellen bezogen wird, bezeichnet die Inkubation in einer wässrigen Zusammensetzung, insbesondere in einem für die betreffenden Zellen geeigneten Nährmedium, unter Bedingungen, die eine metabolische Aktivität der Zellen ermöglichen. Dies setzt insbesondere voraus, dass die wässrige Zusammensetzung alle von den Zellen geforderten Bestandteile enthält, dass der pH in einem für die jeweiligen Zellen akzeptablen Bereich gehalten wird, und dass die Temperatur in einem akzeptablem Bereich, vorzugsweise zwischen 20 °C und 40 °C gehalten wird.

### Verwendung reduzierender Zucker

In noch einer weiteren Ausführungsform betrifft die vorliegende Erfindung die Verwendung reduzierender Zucker in Konzentrationen von wenigstens 10 g/l, bevorzugt wenigstens 20 g/l und stärker bevorzugt wenigstens 30 g/l Gesamtzucker zur Verminderung der Toxizität von AB.

### Verfahren zur Minderung der Toxizität von AB

In noch einer weiteren Ausführungsform betrifft die vorliegende Erfindung ein Verfahren zur Verminderung der Toxizität von AB, enthaltend den Schritt des Kontaktierens freier AB in wässriger Lösung mit einem reduzierenden Zucker. Das molare Verhältnis der Gesamtmenge der Aldehydgruppen des reduzierenden Zuckers zu Gesamt-AB beträgt dabei wenigstens 0,3 : 1,0, bevorzugt wenigstens 0,4 : 1 und noch stärker bevorzugt wenigstens 0,5 : 1.

Alle weiter oben gegebenen Definitionen gelten auch für diese Ausführungsform. Die Definitionen für die wässrige Zusammensetzung gelten auch für die hier genannte wässrige Lösung.

Das Kontaktieren kann auf jede für den Fachmann bekannte Weise erfolgen. In einer bevorzugten Ausführungsform der vorliegenden Erfindung liegt der Zucker gelöst in der wässrigen Lösung, besonders bevorzugt in einem Nährmedium, vor. Das Nährmedium enthält vorzugsweise prokaryotische Zellen, die ein im Nährmedium enthaltenes fermentierbares Substrat zu AB umsetzen. Die von den prokaryotischen Zellen gebildete AB wird von den Zellen ausgeschieden und kommt dann unmittelbar in Kontakt mit dem bereits in Lösung vorliegenden reduzierenden Zucker.

### Verfahren zur Kultivierung einer prokaryotischen Zelle

In einer weiteren bevorzugten Ausführungsform betrifft die vorliegende Erfindung ein Verfahren zur Kultivierung von prokaryotischen Zellen in Anwesenheit von AB und wenigstens eines reduzierenden Zuckers, wobei
a) die Zellen solche des Genus *Corynebacterium* sind und die Gesamtmenge Aminobenzoesäure (AB) wenigstens 40 g/l beträgt; oder
b) die Zellen solche des Genus *Escherichia* oder *Pseudomonas* sind und die Gesamtmenge Aminobenzoesäure (AB) wenigstens 5 g/l beträgt;
und das molare Verhältnis der Gesamtmenge der Aldehydgruppen des reduzierenden Zuckers zu Gesamt-AB wenigstens 0,3 : 1,0 beträgt.

Alle weiter oben gegebenen Definitionen gelten auch für diese Ausführungsform.

Die prokaryotische Zelle ist bevorzugt eine Zelle, die in der Lage ist, ein fermentierbares Substrat zu AB umzusetzen. Besonders bevorzugt ist sie in der Lage, den eingesetzten reduzierenden Zucker hierfür als fermentierbares Substrat zu nutzen. Deswegen handelt es sich bevorzugt um ein Kultivierungsverfahren, bei dem die prokaryotischen Zellen AB bilden, d.h. ein Herstellungsverfahren für AB durch eine mikrobielle Fermentation.

Die Inkubation von mikrobiellen Zellen in einem Medium, in dem ein Teil der dort vorliegenden AB glykiert vorliegt, kann die Toxizität der AB reduzieren. Dies ist für alle mikrobiellen Verfahren relevant, in denen AB im Medium vorliegt. In erster Linie betrifft dies Verfahren zur Herstellung von oAB oder pAB durch mikrobielle Fermentation. In solchen Verfahren reichert sich AB bestimmungsgemäß an und es ist für die Effizienz solcher Verfahren vorteilhaft, wenn die Fermentation möglichst lange, d.h. bis zum Erreichen möglichst hoher AB-Konzentrationen geführt werden kann, ohne die Aktivität der Zellen zu beeinträchtigen. Die Reduzierung der Toxizität von AB durch Glykierung ist aber auch für Verfahren aktiv, bei denen zwar keine AB produziert wird, die Mikroorganismen aber trotzdem mit ihr in Kontakt kommen. Dies sind insbesondere Verfahren, bei denen die AB nicht das gewünschte Produkt ist, sondern der Ausgangsstoff, der durch eine mikrobielle Fermentation zu einem Zielprodukt umgesetzt werden soll. Insgesamt bietet die vorliegende Erfindung immer dann Vorteile, wenn mikrobielle Zellen mit AB in Kontakt kommen und eine Verringerung ihrer Stoffwechselaktivität oder ihrer Lebensfähigkeit unerwünscht ist.

Die nachfolgenden Ausführungsbeispiele dienen nur dazu, die vorliegende Erfindung zu illustrieren. Sie sollen den Schutzbereich der Patentansprüche in keiner Weise beschränken.

### Ausführungsbeispiele

### Beispiel 1: Reaktion von Glucose und oAB zu gly-oAB

Durch Neutralisation mit 5 M NaOH wurden 150 g/L oAB in Wasser gelöst und auf pH 7,0 eingestellt. Separat wurden 120 g/L Glucose in Wasser gelöst und ebenfalls auf pH 7,0 eingestellt. Aus verschiedenen Anteilen dieser beiden Lösungen und Wasser wurden insgesamt 9 unterschiedliche Ansätze hergestellt mit Glucose-Konzentrationen von 10, 30 bzw. 60 g/L und oAB-Konzentrationen von 5, 40 bzw. 75 g/L. Diese Ansätze wurden in Duplikaten in 2 mL-Reaktionsgefäßen hergestellt und in einem ThermoMixer^{®} C (Eppendorf) bei 1000 upm und 33 °C inkubiert. Der Verlauf der Reaktion zwischen Glucose und oAB wurde durch die Bestimmung der noch vorhandenen freien Glucose via Cedex Bio Analyzer (Roche) verfolgt. Dabei entspricht eine Abnahme der gemessenen GlucoseKonzentration in mol/L der Zunahme der gly-oAB-Konzentration in mol/L. Nach ca. 75 h war keine weitere Änderung der Glucose-Konzentration erkennbar.

In Tabelle 1 ist der Anteil von gly-oAB an der insgesamt eingesetzten Menge an oAB nach 75 h für die verschiedenen Ansätze dargestellt. Je nach anfänglicher Glucose- und oAB-Konzentration liegen im Gleichgewicht ca. 8,8 - 87,3 % der insgesamt vorhandenen oAB als glykiertes Molekül vor.

**Tabelle 1: Anteil von gly-oAB an der insgesamt eingesetzten Menge an oAB nach 75 h für verschiedene Startkonzentrationen an Glucose und oAB.**

| **Eingesetzte GlucoseKonzentration [g/L]** | **Eingesetzte ortho-Aminobenzoesäure-Konzentration [g/L]** | **Molarer Anteil gly-oAB/oAB_{ges} nach 75 h [%]** |
|---|---|---|
| 10 | 5 | 16,2 ± 1,9% |
| 10 | 40 | 11,1 ± 0,1 % |
| 10 | 75 | 8,8 ± 0,0 % |
| 30 | 5 | 77,0 ± 1,5 % |
| 30 | 40 | 26,5 ± 0,2 % |
| 30 | 75 | 20,7 ± 0,1 % |
| 60 | 5 | 87,3 ± 5,8 % |
| 60 | 40 | 45,6 ± 0,2 % |
| 60 | 75 | 37,1 ± 0,1 % |

### Beispiel 2: Reaktion von Glucose und pAB zu gly-pAB

Durch Neutralisation mit 5 M NaOH wurden 150 g/L pAB in Wasser gelöst und auf pH 7,0 eingestellt. Separat wurden 120 g/L Glucose in Wasser gelöst und ebenfalls auf pH 7,0 eingestellt. Aus verschiedenen Anteilen dieser beiden Lösungen und Wasser wurden insgesamt 9 unterschiedliche Ansätze hergestellt mit Glucose-Konzentrationen von 10, 30 bzw. 60 g/L und pAB-Konzentrationen von 5, 40 bzw. 75 g/L. Diese Ansätze wurden in Duplikaten in 2 mL-Reaktionsgefäßen hergestellt und in einem ThermoMixer^{®} C (Eppendorf) bei 1000 upm und 33 °C inkubiert. Der Verlauf der Reaktion zwischen Glucose und pAB wurde durch die Bestimmung der noch vorhandenen freien Glucose via Cedex Bio Analyzer (Roche) verfolgt. Dabei entspricht eine Abnahme der gemessenen GlucoseKonzentration in mol/L der Zunahme der gly-pAB-Konzentration in mol/L. Nach ca. 75 h war keine weitere Änderung der Glucose-Konzentration erkennbar.

In Tabelle 2 ist der Anteil von gly-pAB an der insgesamt eingesetzten Menge an pAB nach 75 h für die verschiedenen Ansätze dargestellt. Je nach anfänglicher Glucose- und pAB-Konzentration liegen im Gleichgewicht ca. 7,8 - 83,3 % der insgesamt vorhandenen pAB als glykiertes Molekül vor.

**Tabelle 2: Anteil von gly-pAB an der insgesamt eingesetzten Menge an pAB nach 75 h für verschiedene Startkonzentrationen an Glucose und pAB.**

| **Eingesetzte GlucoseKonzentration [g/L]** | **Eingesetzte para-Aminobenzoesäure-Konzentration [g/L]** | **Molarer Anteil gly-pAB/pAB_{ges} nach 75 h [%]** |
|---|---|---|
| 10 | 5 | 16,1 ± 0,0 % |
| 10 | 40 | 11,1 ± 0,1 % |
| 10 | 75 | 7,8 ± 0,0 % |
| 30 | 5 | 46,1 ± 3,2 % |
| 30 | 40 | 31,2 ± 0,0 % |
| 30 | 75 | 22,6 ± 0,0 % |
| 60 | 5 | 83,3 ± 3,2 % |
| 60 | 40 | 54,2 ± 0,1 % |
| 60 | 75 | 42,0 ± 0,0 % |

### Beispiel 3: Bestimmung von gly-oAB und gly-pAB durch saure Rückreaktion

Wegen der Verstoffwechselung von Glucose in Fermentationsprozessen kann glykierte Aminobenzoesäure nicht über eine Abnahme der Glucosekonzentration quantifiziert werden, da diese Abnahme auch auf Glucoseverbrauch beruhen kann. Unter sauren Bedingungen ist jedoch eine Rückreaktion von gly-oAB oder gly-pAB zu freier Glucose und Aminobenzoesäure möglich. Dies ermöglicht eine Quantifizierung von gly-oAB bzw. gly-pAB über die vor und nach Rückreaktion vorhandene freie Glucosekonzentration, sofern die Rückreaktion annähernd vollständig abläuft.

Um dies zu testen, wurden wie in Bsp. 1 und 2 beschrieben verschiedene Glucose-Aminobenzoesäure-Mischungen für ca. 75 h inkubiert, um glykierte Aminobenzoesäure zu bilden. 200 µL des jeweiligen Ansatzes wurden dann mit 800 µL 1 M Salzsäure gemischt und bei Raumtemperatur stehen gelassen. In regelmäßigen Abständen wurde die freie Glucose via Cedex Bio Analyzer (Roche) gemessen. Nach ca. 2 h war keine weitere Änderung der Glucose-Konzentration erkennbar.

In Tabelle 3 ist der prozentuale Anteil der nach saurer Rückreaktion gemessenen freien Glucose bezogen auf die zu Beginn eingesetzte Glucose-Konzentration für die Rückreaktion von gly-pAB dargestellt. Unabhängig von den eingesetzten Glucose- und pAB-Konzentrationen können mehr als 95,4 % der vorhandenen Glucose nach Säurebehandlung wiedergefunden werden.

**Tabelle 3: Rückreaktion von gly-pAB unter sauren Bedingungen**

| **Eingesetzte GlucoseKonzentration [g/L]** | **Eingesetzte para-Aminobenzoesäure-Konzentration [g/L]** | **Durch saure Rückreaktion wiedergefundene Glucose [%]** |
|---|---|---|
| 10 | 5 | 98,3 ± 0,2 % |
| 10 | 40 | 98,0 ± 0,2 % |
| 10 | 75 | 97,6 ± 0,0 % |
| 30 | 5 | 98,0 ± 0,1 % |
| 30 | 40 | 95,4 ± 1,0 % |
| 30 | 75 | 96,6 ± 0,3 % |
| 60 | 5 | 97,7 ± 0,2 % |
| 60 | 40 | 97,9 ± 0,3 % |
| 60 | 75 | 97,7 ± 0,7 % |

In Tabelle 4 ist der prozentuale Anteil der nach saurer Rückreaktion gemessenen freien Glucose bezogen auf die zu Beginn eingesetzte Glucose-Konzentration für die Rückreaktion von gly-oAB dargestellt. Mit steigender oAB-Konzentration bzw. sinkender Glucose-Konzentration verringert sich der Anteil der vorhandenen Glucose, die nach Säurebehandlung wiedergefunden werden kann. Bei Glucose-Konzentrationen von 30 g/L oder mehr sowie oAB-Konzentrationen bis zu 75 g/L können jedoch mehr als 88,2 % der vorhandenen Glucose wiedergefunden werden. Die saure Rückreaktion kann daher zur Quantifizierung von gly-pAB und mit Einschränkungen gly-oAB genutzt werden.

**Tabelle 4: Rückreaktion von gly-oAB unter sauren Bedingungen**

| **Eingesetzte GlucoseKonzentration [g/L]** | **Eingesetzte ortho-Aminobenzoesäure-Konzentration [g/L]** | **Durch saure Rückreaktion wiedergefundene Glucose [%]** |
|---|---|---|
| 30 | 40 | 96,1 ± 2,4 % |
| 10 | 75 | 63,0 ± 0,3 % |
| 30 | 75 | 88,6 ± 1,4 % |
| 60 | 75 | 88,2 ± 0,7 % |

### Beispiel 4: Demonstration der reduzierten Toxizität von glykierter oAB oder pAB für Mikroorganismen

### Allgemeine Hinweise zum experimentellen Design

Für die nachfolgenden Beispiele wurde das Kleinstkultivierungssystem BioLector Pro von m2p-labs (Aachen, Deutschland) verwendet, welches 48 parallele Kultivierungen in einer Mikrotiterplatte erlaubt. Die Bakterienstämme wurden in initial 800 µL und final 1500 µL Medium in 48-Well-Flowerplates vom Typ BOH2 (m2p-labs, Aachen, Deutschland) bei 1000 rpm (Schüttelauslenkung 3 mm) kultiviert. Die verwendeten Platten enthalten immobilisierte Indikatorsubstanzen (Optoden), über welche eine Online-Messung des pH-Wertes und des Sauerstoffpartialdruckes pO₂ in jedem Well ermöglicht wird. Das Wachstum wurde für alle Kultivierungen bei 620 nm durch Messung des Streulicht-Signals beobachtet (eingestellter "gain" von 3). Das Parallelkultivierungssystem von m2p-labs ist für die Kultivierung von Bakterienstämmen bekannt und geeignet.

Die Kultivierungstemperatur und verwendeten Medien richten sich nach dem untersuchten Bakterienstamm und werden in Tabelle 5 neben weiteren Kultivierungsparametern näher aufgeführt. Für die Beispiele wurden die bakteriellen Wildtypstämme *Corynebacterium glutamicum* ATCC 13032, *Escherichia coli* K12 und *Pseudomonas putida* DSM 6125 ausgewählt. Für *C. glutamicum* wird in der Hauptkultivierung CG XII-Minimalmedium, für *E. coli* und *P. putida* LB-Komplex-Medium (Miller's LB-Bouillon (L3522, Sigma-Aldrich, St. Louis, USA)) eingesetzt, welche typischerweise für die Kultivierungen dieser und gattungsverwandter Spezies verwendet werden. Die genauen Medienzusammensetzungen mit Anpassungen sind in Tabelle 6 näher beschrieben.

**Tabelle 5: Vorkulturführung, verwendete Medien sowie Kultivierungsparameter der nachfolgend gezeigten Beispiele.**

| **Stamm** | | ***Escherichia coli* K12** | ***Pseudomonas putida* DSM 6125** | ***Corynebacterium glutamicum* ATCC 13032** |
|---|---|---|---|---|
| Kultivierungstemperatur | | 37 °C | 28 °C | 30 °C |
| Medium für Vorkultur 1 (über Nacht) | | LB-MOPS | LB-MOPS | SY |
| Medium für Vorkultur 2 (über Nacht) | | LB-MOPS | LB-MOPS | CG XII Hefeextrakt |
| Medium für Hauptkultur | | LB-MOPS | LB-MOPS | CG XII Hauptkultur |
| | Inokulum (OD600) | 0,5 | 1 | 1 |
| | Zeitpunkt des *Spikes* | ca. 4 h | ca. 5-7 h | ca. 7 h |

**Tabelle 6: Medienzusammensetzung und Stammlösungen**

| **Bezeichnung der Medien** | | | **Zusammensetzung** |
|---|---|---|---|
| A | LB-MOPS | | 20 g/L LB Broth Base, 62 g/L MOPS, pH 7 |
| A1 | | LB-MOPS mit freier oAB oder pAB ohne Glucose | 20 g/L LB Broth Base, 62 g/L MOPS, 300 g/L oAB oder pAB, pH 7 (ca. 87 g/L NaOH) |
| A2 | LB-MOPS mit 450 g/L Glucose | | 20 g/L LB Broth Base, 62 g/L MOPS, 450 g/L Glucose, pH 7 |
| A3 | | LB-MOPS mit gly-oAB oder gly-pAB | Mischung aus A1 und A2 im Verhältnis 2:1, unter Rühren mindestens 70 h inkubieren lassen. |
| B | SY | | 10 g/L Hefeextrakt, 16 g/L Sojapepton, 5 g/L NaCl, 10 g/L Glucose |
| C | CG XII Hefeextrakt | | 1 g/L KH₂PO₄, 1 g/L K₂HPO₄, 10 g/L (NH₄)₂SO₄, 42 g/L MOPS, 5 g/L Urea, 5 g/L Hefeextrakt, 20 g/L Glucose, 0,01 g/L CaCl₂, 0,25 g/L MgSO₄, 0,002 g/L Biotin, 1 x Spurenelemente |
| D | CG XII-Hauptkulturmedium mit 2 % Glucose | | 1 g/L KH₂PO₄, 1 g/L K₂HPO₄, 10 g/L (NH₄)₂SO₄, 42 g/L MOPS, 5 g/L Urea, 20 g/L Glucose, 0,01 g/L CaCl₂, 0,25 g/L MgSO₄, 0,002 g/L Biotin, 1 x Spurenelemente |
| D1 | | CG XII mit 300 g/L freier oAB oder pAB ohne Glucose | 1 g/L KH₂PO₄, 1 g/L K₂HPO₄, 10 g/L (NH₄)₂SO₄, 42 g/L MOPS, 5 g/L Urea, 0,01 g/L CaCl₂, 0,25 g/L |
| | | | MgSO₄, 0,002 g/L Biotin, 1 x Spurenelemente, 300 g/L oAB oder pAB, pH 7 (ca. 87,4 g/L NaOH) |
| D2 | CG XII mit 450 g/L Glucose | | 1 g/L KH₂PO₄, 1 g/L K₂HPO₄, 10 g/L (NH₄)₂SO₄, 42 g/L MOPS, 5 g/L Urea, 450 g/L Glucose, 0,01 g/L CaCl₂, 0,25 g/L MgSO₄, 0,002 g/L Biotin, 1 x Spurenelemente |
| D3 | | CG XII mit gly-oAB oder gly-pAB | Mischung aus D1 und D2 im Verhältnis 2:1, unter Rühren mindestens 70 h inkubieren lassen. |
| E | 1000x Spurenelemente | | 10 g/L MnSO₄ x H₂O, 10 g/L FeSO₄ x 7 H₂O, 1 g/L ZnSO₄ x 7 H₂O, 0,2 g/L CuSO₄ x 5 H₂O, 0,02 g/L NiCl₂ x 6 H₂O |

Um die toxischen Effekte von freier oAB bzw. freier pAB im Vergleich zu der glykierten Form gly-oAB bzw. gly-pAB zu bestimmen, wurden nach einer initialen Anwuchsphase weitere Hauptkulturmedien mit unterschiedlicher Konzentration an oAB und gly-oAB bzw. pAB und gly-pAB zugegeben (im Folgenden *Spike* genannt). Die Herstellung von Hauptkulturmedium mit gly-oAB bzw. gly-pAB-Form richtete sich nach der Kinetik in Beispiel 1 und 2 und erfolgte für 70 h unter Rühren. Als Referenz wurden Medien ohne AB zugesetzt (im folgenden Referenz-Spikes genannt).

Da es durch die gly-oAB- oder gly-pAB-Spikes zu einer Erhöhung der zugeführten Kohlenstoffmenge in Form der im Komplex gebundenen sowie noch freien Glucose aus der Gleichgewichtsreaktion kommt, wurde die gleiche Menge an Glucose auch bei den Referenz-Spikes sowie den oAB und pAB-Spikes zugeführt, hier allerdings ohne vorherige Inkubation, so dass sich der Komplex zwischen oAB bzw. pAB und Glucose zum Zeitpunkt der Zugabe noch nicht ausgebildet hatte. Dies wurde experimentell über die in Beispiel 3 gezeigte Methode überprüft.

Die Tabellen 7 (oAB) und 8 (pAB) geben eine Übersicht der experimentellen Ansätze. So ist für jeden Ansatz die Art und Menge der unterschiedlichen nach der Anwuchsphase zugegebenen Lösungen aufgelistet. Die Menge bezieht sich dabei jeweils auf ein einzelnes Kultivierungs-Well der Mikrotiterplatte. Während zu Beginn der Kultivierung 800 µL inokuliertes Medium eingesetzt wurden, wurden jedem Well nach der Anwuchsphase in Summe 700 µL an Lösungen hinzugefügt. Zusätzlich ist die jeweils resultierende AB-Konzentration nach Spike-Zugabe sowie die Erhöhung der GlucoseKonzentration im Well durch den *Spike* angegeben. Die Zusammensetzung und Herstellung der verwendeten Lösungen ist zudem in Tabelle 6 beschrieben.

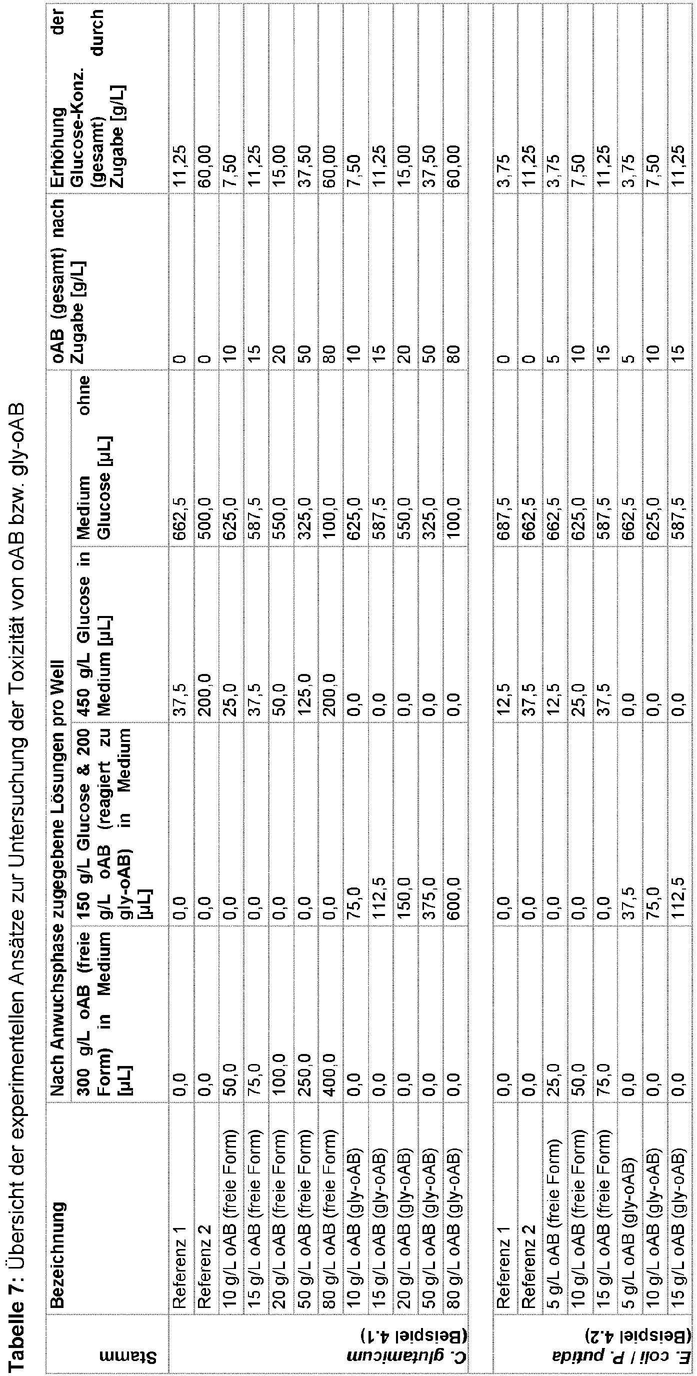

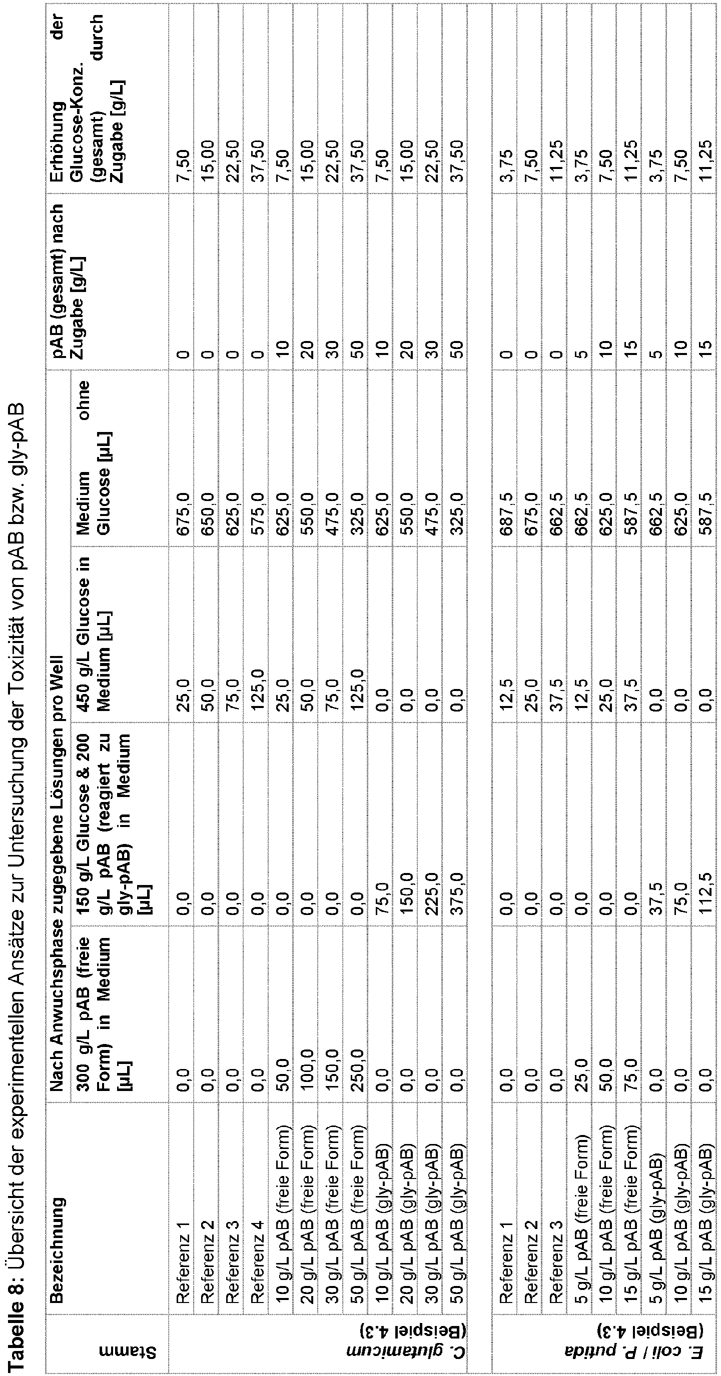

### Beispiel 4.1: Toxizität von gly-oAB gegenüber freier oAB für Corynebacterium glutamicum ATCC 13032

Für das nachfolgende Beispiel wurde der Stamm *Corynebacterium glutamicum* ATCC 13032 in 800 µL Minimalmedium CG XII im Kleinstkultivierungssystem BioLector Pro von m2p-labs wie vorab beschrieben kultiviert. Nach 7,2 h erfolgte die Zugabe unterschiedlicher Konzentrationen an oAB bzw. gly-oAB. Alle Ansätze und Referenzen wurden in Triplikaten durchgeführt (Tabelle 7: Beispiel 4.1).

Die Toxizität von oAB bzw. gly-oAB wurde anhand des Wachstums vergleichend zu 2 Kontrollen ohne oAB/gly-oAB-Spike untersucht (Referenz 1 und Referenz 2). Dazu wurde die Steigung des Streulicht-Signals in den ersten 10 h nach erfolgtem *Spike* analysiert (Tabelle 9). Sowohl oAB als auch gly-oAB führen zu einer Verzögerung des Wachstums. Bei gleicher Konzentration an oAB im oAB- oder gly-oAB-*Spike,* zeigt sich bei allen Konzentrationen jedoch eine geringere Toxizität von gly-oAB gegenüber freier oAB anhand einer höheren Steigung im Streulicht-Signal.

**Tabelle 9: Steigung des Streulicht-Signals einer Kultivierung des Stamms C. glutamicum ATCC 13032 in den ersten 10 h nach Spike mit oAB, gly-oAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 7,57 |
| Referenz 2 | 6,28 |
| 10 g/L oAB (freie Form) | 4,48 |
| 10 g/L oAB (gly-oAB) | 7,17 |
| 15 g/L oAB (freie Form) | 3,89 |
| 15 g/L oAB (gly-oAB) | 5,27 |
| 20 g/L oAB (freie Form) | 3,04 |
| 20 g/L oAB (gly-oAB) | 4,53 |
| 50 g/L oAB (freie Form) | 0,61 |
| 50 g/L oAB (gly-oAB) | 1,38 |
| 80 g/L oAB (freie Form) | -0,67 |
| 80 g/L oAB (gly-oAB) | 0,012 |

### Beispiel 4.2: Toxizität von gly-oAB gegenüber freier oAB für Escherichia coli K12 und Pseudomonas putida DSM 6125

Für das nachfolgende Beispiel wurden die Stämme *Escherichia coli* K12 und *Pseudomonas putida* DSM 6125 in 800 µL LB-Medium im Kleinstkultivierungssystem BioLector Pro von m2p-labs wie vorab beschrieben kultiviert. Nach 4 h *(E. coli)* bzw. 7 h *(P. putida)* erfolgte die Zugabe unterschiedlicher Konzentrationen an oAB bzw. gly-oAB. Alle Ansätze und Referenzen wurden in Triplikaten durchgeführt (Tabelle 7: Beispiel 4.2).

In Tabelle 10 und 11 ist die Steigung des Streulicht-Signals in den ersten 2 h nach dem *Spike* für *E. coli* und in den ersten 10 h für *P. putida* dargestellt. Bei beiden Organismen wirken sowohl oAB als auch gly-oAB wachstumsinhibierend/toxisch im Vergleich zu den Referenzen. Bei *E. coli* sind bei niedrigster oAB-Konzentration (5 g/L) die Wachstumseinbußen von oAB und gly-oAB ähnlich. Bei 10 und 15 g/L oAB zeigt sich jedoch eine geringere Toxizität von gly-oAB gegenüber freier oAB anhand der höheren Steigung im Streulicht-Signal. Bei *P. putida* zeigt sich bereits bei niedrigster Konzentration (5 g/L) eine geringere Toxizität von gly-oAB gegenüber freier oAB anhand der Steigung des Streulicht-Signals. Bei 10 g/L freier oAB findet kein Wachstum nach dem *Spike* statt, während die Zellen bei gleicher Konzentration an gly-oAB weiter wachsen. Bei höchster Konzentration (15 g/L) findet weder bei freier oAB noch bei gly-oAB Wachstum nach dem *Spike* statt.

Die Verringerung der toxischen Wirkung durch Glykierung von oAB lässt sich also nicht nur für C. *glutamicum* ATCC 13032, sondern auch für *E. coli* K12 und *P. putida* DSM 6125 zeigen.

**Tabelle 10: Steigung des Streulicht-Signals einer Kultivierung des Stamms Escherichia coli K12 in den ersten 2 h nach Spike mit oAB, gly-oAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 4,49 |
| Referenz 2 | 5,02 |
| Referenz 3 | 4,88 |
| 5 g/L oAB (freie Form) | 2,05 |
| 5 g/L oAB (gly-oAB) | 2,05 |
| 10 g/L oAB (freie Form) | 0,94 |
| 10 g/L oAB (gly-oAB) | 1,40 |
| 15 g/L oAB (freie Form) | 0,39 |
| 15 g/L oAB (gly-oAB) | 0,96 |

**Tabelle 11: Steigung des Streulicht-Signals einer Kultivierung des Stamms Pseudomonas putida DSM 6125 in den ersten 10 h nach Spike mit oAB, gly-oAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 2,48 |
| Referenz 3 | 2,64 |
| 5 g/L oAB (freie Form) | 0,36 |
| 5 g/L oAB (gly-oAB) | 0,81 |
| 10 g/L oAB (freie Form) | -0,08 |
| 10 g/L oAB (gly-oAB) | 0,63 |
| 15 g/L oAB (freie Form) | -0,10 |
| 15 g/L oAB (gly-oAB) | -0,12 |

### Beispiel 4.3: Toxizität von gly-pAB gegenüber freier pAB für Corynebacterium glutamicum ATCC 13032, Escherichia coli K12 und Pseudomonas putida DSM 6125

Die Toxizität von pAB und gly-pAB wurde wie vorab in Beispiel 4.1 und 4.2 für oAB und gly-oAB beschrieben und wie in Tabelle 8 dargestellt untersucht. Die Ergebnisse sind in den Tabellen 12, 13 und 14 zusammengefasst. Gezeigt wird hier die Steigung im Streulicht-Signal nach dem *Spike* für alle drei Stämme.

In allen Fällen zeigen sich Wachstumseinbußen bei Zugabe von freier pAB oder gly-pAB verglichen mit den Referenz-Kultivierungen ohne pAB-Zugabe. Für C. *glutamicum* ATCC 13032, zeigte sich bei allen Bedingungen die verminderte Toxizität von gly-pAB gegenüber pAB in freier Form anhand der Steigung des Streulicht-Signals (Tabelle 12) analog zu vorherigen Daten zu gly-oAB und oAB (Tabelle 9).

**Tabelle 12: Steigung des Streulicht-Signals einer von Kultivierung des Stamms Corynebacterium glutamicum ATCC 13032 in den ersten 4,7 h nach Spike mit pAB, gly-pAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 7,68 |
| Referenz 2 | 7,10 |
| Referenz 3 | 6,53 |
| Referenz 4 | 5,17 |
| 10 g/L pAB (freie Form) | 4,47 |
| 10 g/L pAB (gly-pAB) | 5,22 |
| 20 g/L pAB (freie Form) | 2,38 |
| 20 g/L pAB (gly-pAB) | 3,56 |
| 30 g/L pAB (freie Form) | 1,34 |
| 30 g/L pAB (gly-pAB) | 2,39 |
| 50 g/L pAB (freie Form) | 0,40 |
| 50 g/L pAB (gly-pAB) | 0,97 |

Für *E. coli* K12 zeigte sich bei einer Zugabe von 5 g/L pAB nach 4 h kein Vorteil von gly-pAB gegenüber pAB in freier Form. Da die Glukose im pAB-Spike in ungebundener Form vorliegt und damit für den Mikroorganismus zugänglich und als C-Quelle verwertbar ist, ist eine höhere Steigung bei pAB-Spike gegenüber gly-pAB-Spike zu erkennen. Bei 10 und 15 g/L wird der geringere toxische Effekt der glykierten Form anhand der höheren Steigung des Streulicht-Signals verglichen mit dem pAB-Spike deutlich.

**Tabelle 13: Steigung des Streulicht-Signals einer von Kultivierung des Stamms Escherichia coli K12 in den ersten 4 h nach Spike mit pAB, gly-pAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 3,98 |
| Referenz 2 | 5,15 |
| Referenz 3 | 5,06 |
| 5 g/L pAB (freie Form) | 2,02 |
| 5 g/L pAB (gly-pAB) | 1,51 |
| 10 g/L pAB (freie Form) | 1,03 |
| 10 g/L pAB (gly-pAB) | 1,45 |
| 15 g/L pAB (freie Form) | 0,02 |
| 15 g/L pAB (gly-pAB) | 0,67 |

Für *P. putida* DSM 6125 zeigt sich bei einer Zugabe von 5 und 10 g/L pAB nach 5 h kein Unterschied zwischen pAB und gly-pAB. Bei 15 g/L wird der geringere toxische Effekt der glykierten Form anhand der höheren Steigung des Streulicht-Signals verglichen mit dem pAB-Spike jedoch ersichtlich.

**Tabelle 14: Steigung des Streulicht-Signals einer von Kultivierung des Stamms Pseudomonas putida DSM 6125 in den ersten 8 h nach Spike mit pAB, gly-pAB oder einer Mischung aus Medium und Glucose-Lösung (Referenz).**

| **Bezeichnung** | **Steigung des Streulicht-Signals nach Zugabe [a.u./h]** |
|---|---|
| Referenz 1 | 2,14 |
| Referenz 2 | 2,24 |
| Referenz 3 | 2,14 |
| 5 g/L pAB (freie Form) | 0,83 |
| 5 g/L pAB (gly-pAB) | 0,76 |
| 10 g/L pAB (freie Form) | 0,50 |
| 10 g/L pAB (gly-pAB) | 0,51 |
| 15 g/L pAB (freie Form) | 0,07 |
| 15 g/L pAB (gly-pAB) | 0,38 |

## Patentansprüche

1. Wässrige Zusammensetzung enthaltend
a) eine Gesamtmenge Aminobenzoesäure (AB) von wenigstens 40 g/l und mehrere Zellen des Genus *Corynebacterium;*
b) eine Gesamtmenge Aminobenzoesäure (AB) von wenigstens 5 g/l und mehrere Zellen des Genus *Escherichia* oder *Pseudomonas;*
wobei wenigstens 2 g/l dieser Gesamtmenge als glykierte AB (gly-AB) vorliegen.

2. Die wässrige Zusammensetzung nach Anspruch 1, wobei wenigstens 15 % der Gesamtmenge an AB als glykierte AB vorliegen.

3. Die wässrige Zusammensetzung nach Anspruch 1 oder 2, zusätzlich enthaltend wenigstens einen reduzierenden Zucker, wobei das molare Verhältnis der Gesamtmenge der Aldehydgruppen des reduzierenden Zuckers zu Gesamt-AB wenigstens 0,3 : 1,0 beträgt.

4. Die wässrige Zusammensetzung nach Anspruch 3, enthaltend wenigstens einen reduzierenden Zucker, der von den in der wässrigen Zusammensetzung enthaltenen Zellen nicht als Energie- oder Kohlenstoffquelle oder zur Bildung eines Zielprodukts verwertet werden kann.

5. Die wässrige Zusammensetzung nach einem der Ansprüche 1 bis 4, zusätzlich enthaltend eine für die Zellen verwertbare Stickstoffquelle, eine für die enthaltenen Zellen verwertbare Schwefelquelle und eine für die enthaltenen Zellen verwertbare Phosphorquelle.

6. Die wässrige Zusammensetzung nach einem der Ansprüche 1 bis 5, wobei die Zellen in einer Konzentration von 2 g bis 70 g Trockenmasse pro Liter vorliegen.

7. Die wässrige Zusammensetzung nach einem der Ansprüche 1 bis 6, wobei die Zellen metabolisch aktiv sind.

8. Verwendung der wässrigen Zusammensetzung nach einem der Ansprüche 1 bis 6 zur Kultivierung der darin enthaltenen Zellen.

9. Verfahren zur Verminderung der Toxizität von AB enthaltend den Schritt des Kontaktierens freier AB in wässriger Lösung mit einem reduzierenden Zucker, wobei das molare Verhältnis der Gesamtmenge der Aldehydgruppen des reduzierenden Zuckers zu Gesamt-AB wenigstens 0,3 : 1,0 beträgt.

10. Das Verfahren nach Anspruch 9, wobei die zu entgiftende freie AB in der wässrigen Lösung durch die in der wässrigen Zusammensetzung enthaltenen Zellen erzeugt wird.

11. Die Verwendung reduzierender Zucker in Konzentrationen von wenigstens 10 g/l Gesamtzucker zur Verminderung der Toxizität von AB.

12. Die Verwendung nach Anspruch 11, wobei das molare Verhältnis der Gesamtmenge der Aldehydgruppen von reduzierendem Zucker zu Gesamt-AB wenigstens 0,3 : 1,0 beträgt.

13. Verfahren zur Kultivierung von prokaryotischen Zellen in Anwesenheit von AB und wenigstens eines reduzierenden Zuckers, wobei
a) die Zellen solche des Genus *Corynebacterium* sind und die Gesamtmenge Aminobenzoesäure (AB) wenigstens 40 g/l beträgt; oder
b) die Zellen solche des Genus *Escherichia* oder *Pseudomonas* sind und die Gesamtmenge Aminobenzoesäure (AB) wenigstens 5 g/l beträgt;
und das molare Verhältnis der Gesamtmenge der Aldehydgruppen des reduzierenden Zuckers zu Gesamt-AB wenigstens 0,3 : 1,0 beträgt.

14. Das Verfahren nach Anspruch 13, wobei die Zellen in der Lage sind, ein fermentierbares Substrat zu AB umzusetzen.

15. Das Verfahren nach Anspruch 14, wobei die Zellen in der Lage sind, den reduzierenden Zucker als fermentierbares Substrat zu benutzen.

16. Das Verfahren nach Anspruch 13 oder 14, wobei wenigstens ein reduzierender Zucker anwesend ist, der von den prokaryotischen Zellen nicht als Energie- oder Kohlenstoffquelle verwertet werden kann.
